# EUROPEAN PATENT APPLICATION

(11) **EP 4 082 553 A1**
(43) Date of publication of application: **02.11.2022**
(21) Application number: 21861965.8
(22) Date of filing: 18.08.2021
(51) Int. Cl.: A61K 35/20, A61K 31/7105, A61P 3/00, A61P 3/04, A61P 9/10, A61P 1/16, A23L 33/10, C12N 5/077

(54) **COMPOSITION FOR INDUCING BROWNING, CONTAINING MILK EXOSOMES**

(30) Priority: 31.08.2020 KR 20200110132
(71) Applicant: UNIVERSITY-INDUSTRY COOPERATION GROUP OF KYUNG HEE UNIVERSITY, Yongin-si, Gyeonggi-do 17104 (KR)
(72) Inventor: KIM, Sang Hoon, Seoul 07002 (KR); BAE, Inseon, Seoul 02447 (KR)
(74) Representative: Isarpatent
(86) International application number: PCT/KR2021/010968
(87) International publication number: WO 2022/045668

(57) **Abstract**

The present invention relates to a composition for inducing browning, a pharmaceutical composition for preventing or treating metabolic diseases, and a food composition for alleviating metabolic diseases, all of the compositions containing milk exosomes. In addition, the present invention relates to a method for inducing the differentiation of white adipocytes into beige adipocytes or brown adipocytes by treatment with the milk exosomes, and a method for treating obesity or metabolic diseases by administering the milk exosomes.

## Description

### TECHNICAL FIELD

The present invention is related to a composition for inducing browning, a pharmaceutical composition for preventing or treating metabolic diseases, and a food composition for alleviating metabolic diseases, all of the compositions containing milk exosomes. In addition, the present invention relates to a method for inducing the differentiation of white adipocytes into beige adipocytes or brown adipocytes by treatment with the milk exosomes, and a method for treating obesity or metabolic diseases by administering the milk exosomes.

### BACKGROUND ART

Recently, as the obesity problem has become more serious, there is widespread recognition that obesity itself is a disease. Whether or not obesity progresses may be typically determined by determining whether or not a person is overweight, and specifically, it may be determined as an increase in body fat at a specific site (for example, abdominal obesity, and the like). When classified as obese, that is, obese people have been reported to have a higher prevalence and mortality rate than other diseases, and have a 4-fold higher mortality rate due to diabetic disease, a 2-fold higher mortality rate due to liver cirrhosis, a 1.6-fold higher mortality rate due to cerebrovascular disease, and a 1.8-fold higher mortality rate due to coronary artery disease, compared to people with normal weight.

Obesity is a serious chronic syndrome with a variety of causes that are characterized by excessive fat accumulation. There are two main goals in treating obesity, the first is to lose weight by burning excess fat, and the second is to ameliorate a metabolic imbalance. Patients showing abdominal obesity are often associated with pathological conditions such as X-syndrome (insulin resistance, type 2 diabetes, hypertension and lipid metabolism disorder), and abdominal obesity acts as a strong risk factor for early arteriosclerosis, ischemic heart disease and cerebrovascular disease.

There is a further need for research and development for the regulation of the excess energy accumulated by adipocytes as obesity and metabolic diseases caused by fat accumulation increase.

### DESCRIPTION OF EMBODIMENTS

### TECHNICAL PROBLEM

The technical problem to be solved by the present invention is to provide a composition for inducing browning, including milk exosomes.

Another technical problem that the present intervention addresses is to provide a pharmaceutical composition for preventing or treating obesity or a metabolic disease, including milk exosomes.

Another technical problem that the present intervention addresses is to provide a method for treating obesity or a metabolic disease, the method including administering milk exosomes to an individual.

Another technical problem to be solved by the present invention is to provide a food composition for ameliorating obesity or a metabolic disease, including milk exosomes.

Finally, another technical problem to be solved by the present invention is to provide a method for inducing differentiation of white adipocytes into beige adipocytes or brown adipocytes, the method including treating white adipocytes with milk exosomes.

### TECHNICAL SOLUTION

An aspect of the present invention to achieve the aforementioned objects related to a composition for inducing browning, including milk exosomes.

In the present invention, "milk exosome" refers to a vesicle of a lipid bilayer derived from milk. The exosomes contain unique proteins, nucleic acids and the like derived from milk, and are biological nanoparticles with a size of 30 to 200 nm, and information consisting of DNA, RNA, peptides, and the like is contained therein. Exosomes affect the microenvironment around cells by safely transporting internal materials from lyases in body fluids, and the like to transmit information to adjacent cells or distant cells. In particular, milk-derived milk exosomes may be used as food because not only a large amount of exosomes are extracted, but also there is an advantage in that they can be stored in a stable state for a long time.

As for the *in vivo* function of adipocytes, adipocytes are cells that store the energy required by the body, and triglyceride is degraded and used, when needed. Adipose tissue is known to play a role not only in energy storage but also in regulating body energy metabolism including fat metabolism and glucose metabolism as an endocrine organ. Further, mesenchymal precursor cells differentiate into adipocytes via preadipocytes, adipocytes accumulate fat in the body through morphological and biochemical changes during the differentiation process, and adipose tissue is increased in size and differentiates from new preadipocytes.

Although the size in such adipocytes can be generally controlled by dietary regulation, the process by which new preadipocytes differentiate into adipocytes is not affected by dietary regulation, so it is important to regulate the differentiation process of adipocytes to control the fundamental treatment or suppression of obesity.

There are white fat, beige fat, and brown fat in the body, and white fat is a fat that remains and is stored in cells after glucose and fatty acids ingested through food are used as an energy source. White fat is usually a source of energy for the body in emergencies and plays a role in absorbing physical impact, but an excessive amount of white fat induces obesity, diabetes, and the like.

'Brown fat' refers to brownish fat that burns white fat as energy and generates heat in the burning process to maintain body temperature. However, there is a limitation that only some adults have brown fat.

Although it is difficult to make brown fat that is not in the body, beige fat may be activated to induce a function like brown fat to appear. Beige fat is a fat that most adults have, and by inducing browning, it may induce the treatment and amelioration of metabolic diseases such as obesity and diabetes by inducing browning to allow the burning of accumulated white fat to appear.

In the present invention, 'browning' all includes inducing beige adipocytes and/or brown adipocytes which are new forms of adipocytes by inducing browning of white fat, and increasing the metabolic activity thereof. In addition, browning includes the generation of a fat burning effect by UCP1 by turning white fat into brown fat.

In an exemplary embodiment of the present invention, it was confirmed that when white adipocytes were treated with milk exosomes, an effect of inducing browning was shown, and thus adipocytes differentiated into beige adipocytes to express beige adipocyte-specific genes (FIG. 5). Furthermore, it was confirmed that when both preadipocytes and fat-derived stem cells were treated with milk exosomes, the expression of C/EBPβ and PRDM16 was increased (FIG. 23), and when inguinal white adipose tissue (iWAT), which is a fat tissue, was also treated with milk exosomes, the browning of white fat was induced by increasing the expression of C/EBPβ and PRDM16 (FIG. 25B).

Specifically, the milk exosomes may include one or more miRNAs selected from the group consisting of miR-11987, miR-122 and miR-11980.

More specifically, the miR-11987 may include a base sequence of SEQ ID NO: 35, the miR-122 may include a base sequence of SEQ ID NO: 36, and the miR-11980 may include a base sequence of SEQ ID NO: 37.

Further, specifically, the milk exosomes may be derived from cows.

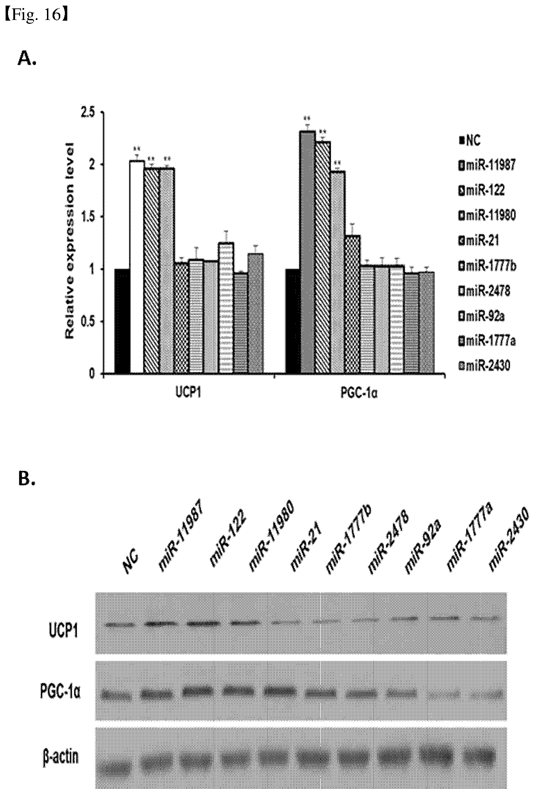

In an exemplary embodiment of the present invention, microRNA present in milk exosomes was confirmed, and it was confirmed that, in particular, when miR-11987, miR-122 and/or miR-11980 are/is overexpressed, the expression of UCP1 and PGC-la was increased (FIG. 16). Accordingly, browning may be induced through milk exosomes including one or more miRNAs selected from the group consisting of miR-11987, miR-122 and miR-11980.

In addition, specifically, the milk exosomes may be characterized by increasing the expression of uncoupling protein-1 (UCP-1) or peroxisome proliferator-activated receptor gamma coactivator 1-alpha (PGC-1α).

The uncoupling protein-1 (UCP-1) increases thermogenesis while being activated in brown fat, and is involved in the promotion of energy consumption by increasing the fat oxidation rate in muscle, which is another organ that induces thermogenesis.

The peroxisome proliferator-activated receptor gamma coactivator1-alpha (PGC-1α) binds to PPAR-α and PPAR-β to increase the expression of genes associated with fatty acid oxidation, and binds to an estrogen receptor related receptor (ERR), which is a nuclear receptor, to reduce glucose oxidation. Furthermore, the PGC-la binds to a transcription factor nuclear respiratory factor 1 (NRF-1) to increase the transcription of OXPHOS-related genes, and increases mitochondrial production by promoting mitochondrial proliferation and DNA transcription.

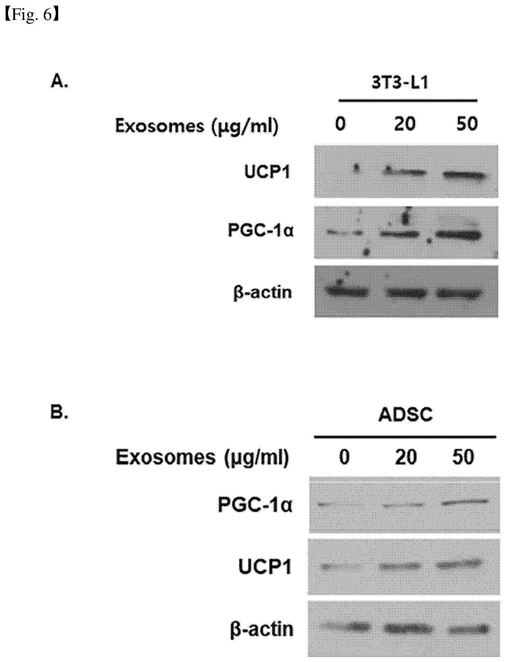

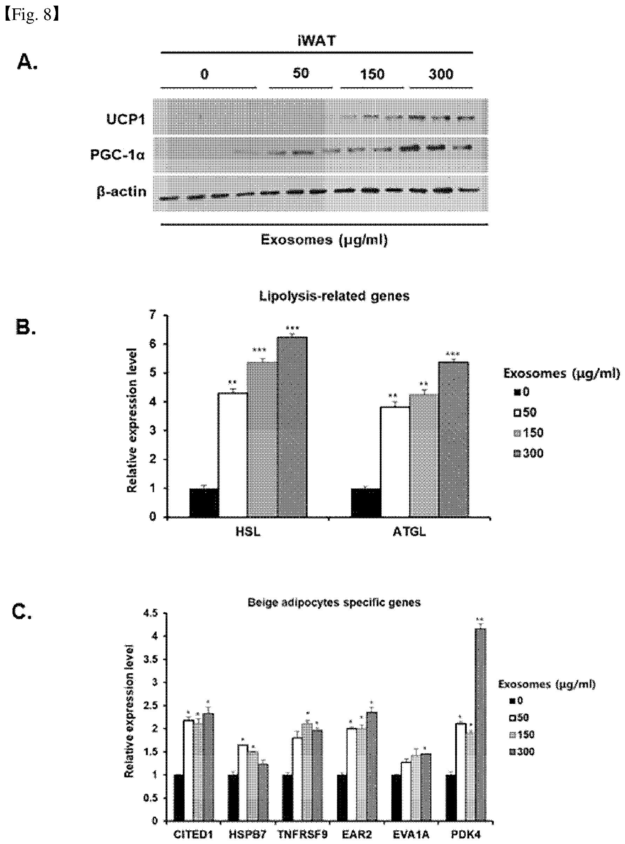

In an exemplary embodiment of the present invention, it was confirmed that the expression of UCP1 and PGC-la proteins associated with thermogenesis was increased in preadipocytes and adipose-derived stem cells during treatment with milk exosomes (FIG. 6), and it was confirmed that the expression of UCP1 and PGC-la proteins was also increased in adipose tissue in obesity-induced mice during treatment with milk exosomes (FIG. 8A).

That is, the milk exosome of the present invention may promote thermogenesis by increasing the expression of UCP1 while inducing browning, and may allow accumulated fat to be burnt by activating metabolism in the body.

Another aspect of the present invention relates to a pharmaceutical composition for preventing or treating obesity or a metabolic disease, including milk exosomes.

In the present invention, obesity means that a portion remaining after most of the ingested calories are consumed is converted into adipocytes and accumulated in the subcutaneous tissue and abdominal cavity in the body. Since there is a limit in the size of adipocytes, the number of adipocytes is increased when excessive energy is ingested, and biomodulators such as hormones, growth factors, and cytokines are affected by the thus-accumulated adipocytes, resulting in metabolic disorders, metabolic diseases, and the like.

Existing anti-obesity agents include thiazolidinediones (TZDs), which inhibit sugar production, sibutramine, which suppresses fat absorption, and the like, but it has been reported that these are originally antidepressants, and cause side effects by causing damage to the cardiovascular system, the central nervous system, the liver, the kidneys, and the like. The milk exosome of the present invention can exhibit an anti-obesity effect, and has reduced side effects and toxicity as a naturally derived material.

In the present invention, a "metabolic disease" is also referred to as a metabolism syndrome and a metabolic syndrome. Metabolic disorders are induced by a living environment, excessive nutrition, insufficient energy consumption, and the like, and obesity occurring while energy and/or fat in the body are/is accumulated causes metabolic diseases.

Specifically, the metabolic disease may be one or more selected from the group consisting of diabetes, hyperlipidemia, hypercholesterolemia, arteriosclerosis and fatty liver, but is not limited thereto. Further, the metabolic disease may also include all metabolic disorders caused by fat accumulation.

The metabolic disease may be ameliorated by facilitating metabolism in the body and burning fat accumulated in the body.

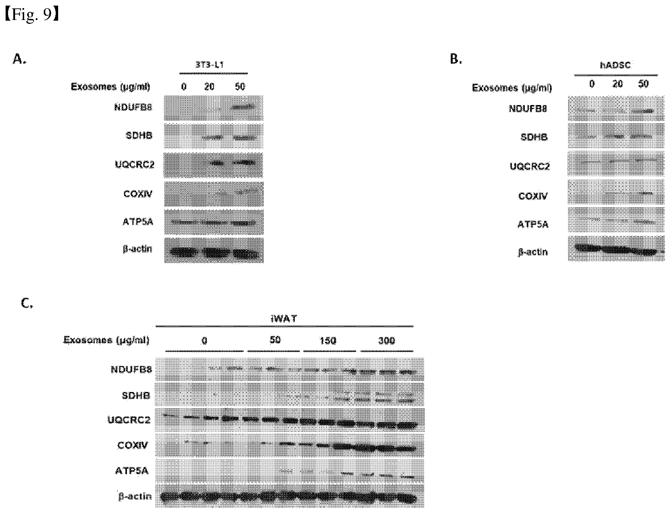

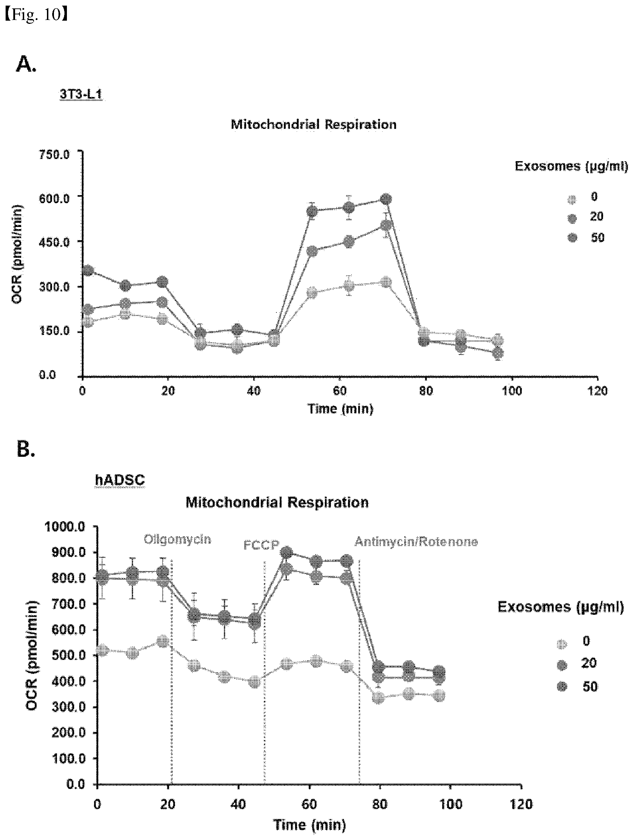

In an exemplary embodiment of the present invention, it was confirmed that mitochondrial activity was promoted during treatment with milk exosomes, and specifically, it was confirmed that the expression of NADH:ubiquinone oxidoreductase subunit B8 (NDUFB8), succinate dehydrogenase complex iron sulfur subunit B (SDHB), ubiquinol-cytochrome C reductase core protein 2 (UQCRC2), cytochrome c oxidase subunit IV (COX IV) and ATP synthase F1 subunit alpha, mitochondrial (ATP5A), which are electron transport chain-related proteins, was increased (FIG. 9), and the oxygen consumption rate was increased (FIG. 10).

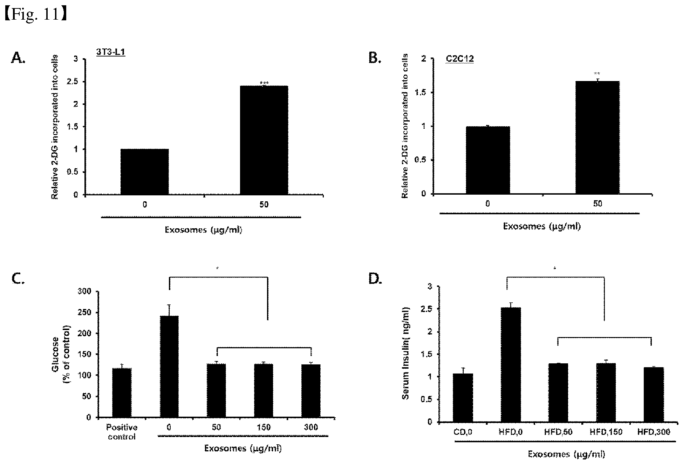

In addition, it was confirmed that during treatment with milk exosomes, blood glucose absorption into cells was increased in both adipocytes and muscle cells (FIGS. 11A and 11B), and blood glucose production was decreased in primary hepatocytes (FIG. 11C). Furthermore, since it was confirmed that serum insulin was decreased during treatment with milk exosomes (FIG. 11D), the milk exosome of the present invention may improve insulin metabolism.

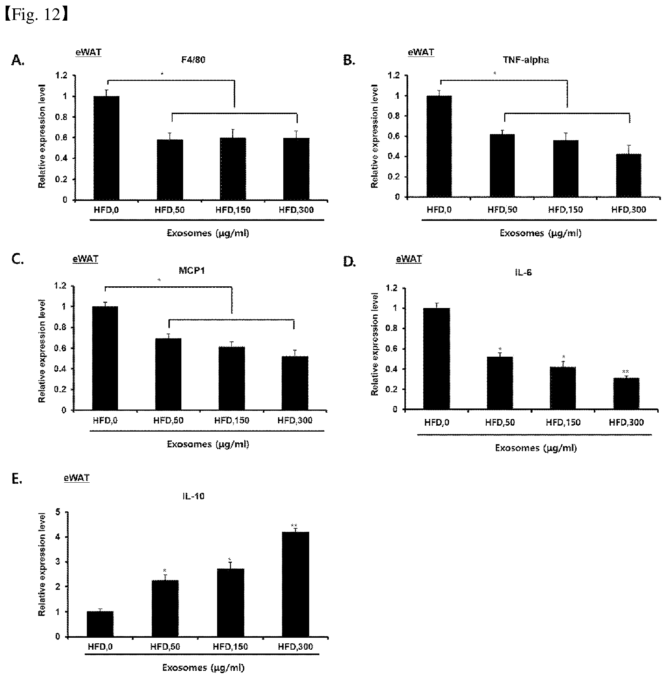

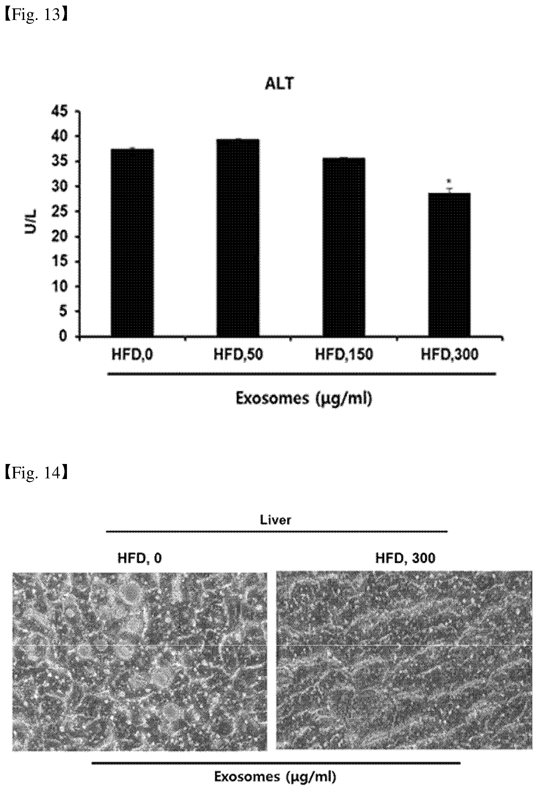

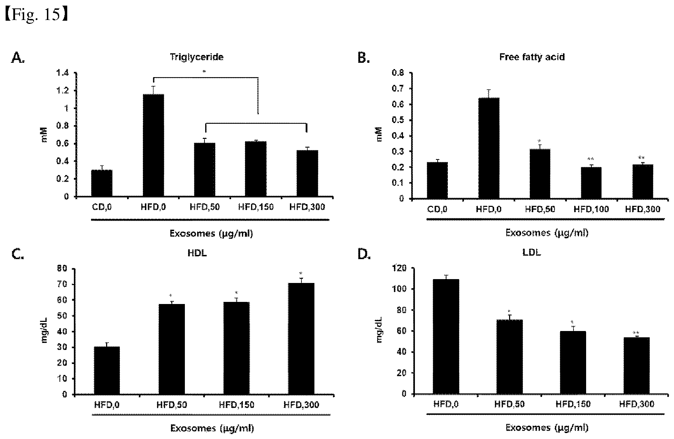

Further, when obesity model mice were treated with milk exosomes, it was confirmed that the expression of inflammatory factors was decreased and the expression of anti-inflammatory factors was increased (FIG. 12), and it was confirmed that the level of ALT, which is a marker factor for fatty liver, was decreased (FIG. 13), triglyceride (FIG. 15A), free fatty acid (FIG. 15B), and LDL (FIG. 15D) were significantly decreased, and HDL (FIG. 15C) was significantly increased.

As described above, since it was confirmed that the milk exosome of the present invention exhibited the effect of mitochondrial activity, the effect of ameliorating insulin resistance and the effect of ameliorating metabolic dysfunction due to obesity, the milk exosome of the present invention may be used for the treatment of obesity and/or a metabolic disease.

Specifically, the milk exosomes may include one or more miRNAs selected from the group consisting of miR-11987, miR-122 and miR-11980.

More specifically, the miR-11987 may include a base sequence of SEQ ID NO: 49, the miR-122 may include a base sequence of SEQ ID NO: 50, and the miR-11980 may include a base sequence of SEQ ID NO: 51.

For administration, the pharmaceutical composition of the present invention may include a pharmaceutically acceptable carrier, excipient or diluent in addition to the milk exosome of the present invention. Examples of the carrier, the excipient or the diluent include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

Further, the pharmaceutical composition of the present invention can be applied in any dosage form, and more specifically, it may be a formulation for parenteral use. The formulation for parenteral use may be in the form of an injection, an application, a spray, such as an aerosol. More specifically, it may be in the form of an injection.

Examples of a preparation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

Still another aspect of the present invention relates to a method for treating obesity or a metabolic disease, the method including administering a pharmaceutically effective amount of milk exosomes to an individual in need of treatment. 'Obesity' and 'metabolic disease' are as described above.

In the present invention, "pharmaceutically effective amount" means an amount sufficient to treat diseases at a reasonable benefit/risk ratio applicable to medical treatment, and an effective dosage level may be determined according to factors including the sexually transmitted disease, age, and type of disease of a patient, the severity of disease, the activity of drugs, sensitivity to drugs, administration time, administration route, excretion rate, treatment period, and simultaneously used drugs, and other factors well known in the medical field.

The pharmaceutical composition of the present invention may be administered as an individual therapeutic agent or may be administered in combination with other therapeutic agents, and may be administered sequentially or simultaneously with therapeutic agents in the related field. In addition, the pharmaceutical composition of the present invention may be administered in a single dose or multiple doses. It is important to administer the composition in a minimum amount that can obtain the maximum effect without any side effects, in consideration of all the aforementioned factors, and this amount may be easily determined by the person skilled in the art.

The term "individual" of the present invention includes animals or humans having a metabolic disease whose symptoms can be ameliorated by administration of the pharmaceutical composition according to the present invention. By administering the therapeutic composition according to the present invention to an individual, a metabolic disease may be effectively prevented and treated.

As used herein, the term "administration" refers to introduction of a predetermined material to a human or animal by any appropriate method, and for the route of administration of the therapeutic composition according to the present invention, the therapeutic composition of the present invention may be orally or parenterally administered via any general route, which may reach a target tissue. Furthermore, the therapeutic composition according to the present invention may be administered by any device which may allow an active ingredient to move to a target cell.

A preferred dosage of the pharmaceutical composition according to the present invention varies depending on the condition and body weight of a patient, the degree of a disease, the form of drug, the administration route, and the duration, but may be appropriately selected by a person skilled in the art.

Yet another aspect of the present invention relates to a food composition for ameliorating obesity or a metabolic disease, including milk exosomes.

The type of food is not particularly limited. Foods to which the milk exosome of the present invention may be added include sausage, meat, bread, chocolate, snacks, candy, confectioneries, ramen, pizza, other noodles, gum, dairy products including ice cream, various soups, beverages, tea, drinks, alcoholic beverages, and vitamin complexes. When formulated as a beverage, the liquid component added in addition to the milk exosome of the present invention is not limited thereto, but may contain various flavoring agents or natural carbohydrates as additional components as in a typical beverage. The above-described natural carbohydrates may be monosaccharides (for example, glucose, fructose, and the like), disaccharides (for example, maltose, sucrose, and the like) and polysaccharides (for example, typical sugars such as dextrin and cyclodextrin), and a sugar alcohol such as xylitol, sorbitol and erythritol.

The type of food may specifically be a health functional food. The health functional food may contain various nutritional supplements, vitamins, minerals (electrolytes), flavoring agents such as synthetic flavoring agents and natural flavoring agents, colorants and enhancers (cheese, chocolate, and the like), pectic acid and salts thereof, organic acids, protective colloid thickeners, pH adjusting agents, stabilizers, preservatives, glycerin, alcohol, carbonating agents used in a carbonated beverage, and the like. In addition, the health functional food of the present invention may contain the pulp for the preparation of fruit and vegetable beverages. These ingredients may be used either alone or in combination thereof.

The health functional food is a food that emphasizes the bioregulatory function of food, and is a food with added value to act for and express a specific purpose using a physical, biochemical, and bioengineering method. The ingredients of these health functional foods are designed and processed to sufficiently exert the body control functions related to the biological defense, regulation of body rhythms, and prevention and recovery of diseases for organisms and may contain food supplement additives, sweeteners or functional raw materials that are acceptable as food.

When the milk exosome of the present invention is used as health functional foods (or health functional beverage additives), the milk exosome is added as it is or used with other foods or food ingredients, and may be appropriately used according to a typical method. The mixing amount of the milk exosome may be suitably determined according to the purpose of their use (prevention, health or improvement, therapeutic treatment).

Yet another aspect of the present invention relates to a method for inducing differentiation of white adipocytes into beige adipocytes or brown adipocytes, the method including treating white adipocytes with milk exosomes.

Specifically, the milk exosomes may include one or more miRNAs selected from the group consisting of miR-11987, miR-122 and miR-11980.

More specifically, the miR-11987 may include a base sequence of SEQ ID NO: 49, the miR-122 may include a base sequence of SEQ ID NO: 50, and the miR-11980 may include a base sequence of SEQ ID NO: 51.

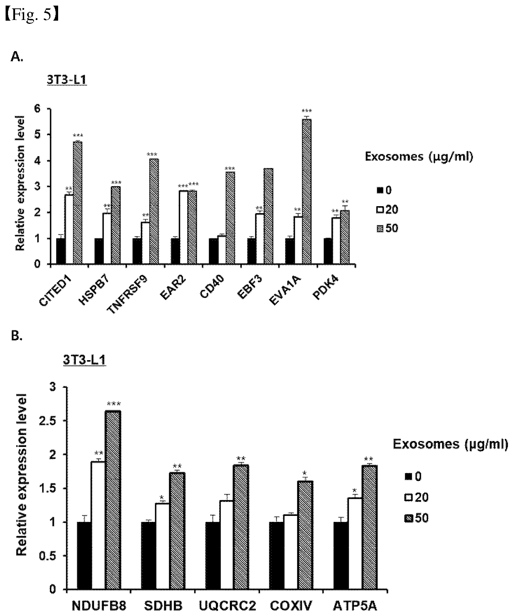

In an exemplary embodiment of the present invention, since it was confirmed that treatment with milk exosomes exhibited a browning-inducing effect (FIGS. 5, 23 and 25), the differentiation of white fat into beige fat or brown fat may be induced by using the milk exosome of the present invention to promote browning.

### ADVANTAGEOUS EFFECTS OF INVENTION

The composition including the milk exosome of the present invention increases the calorie consumption of adipocytes by promoting the generation of heat in the body while inducing browning of white adipocytes to increase energy consumption.

Further, the milk exosome of the present invention is a natural material and has excellent biocompatibility because it is not toxic, and thus can be widely used for the treatment of obesity or a metabolic disease.

The effect of the present invention is not limited to the aforementioned effects, and it should be understood to include all possible effects deduced from the configuration of the invention described in the detailed description or the claims of the present invention.

### BRIEF DESCRIPTION OF THE DRAWINGS

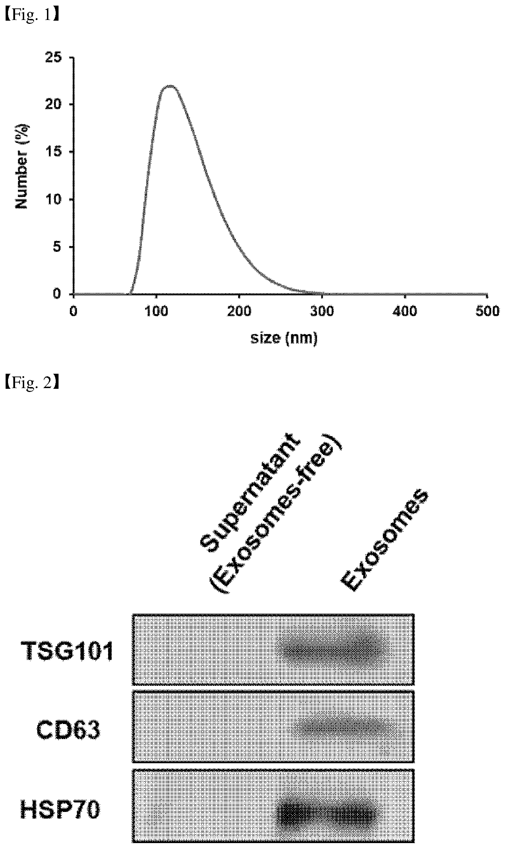

FIG. 1 shows the results of confirming the size of milk exosomes.
FIG. 2 shows the results of confirming the expression of milk exosome marker proteins TSG101, CD9 and HSP70.

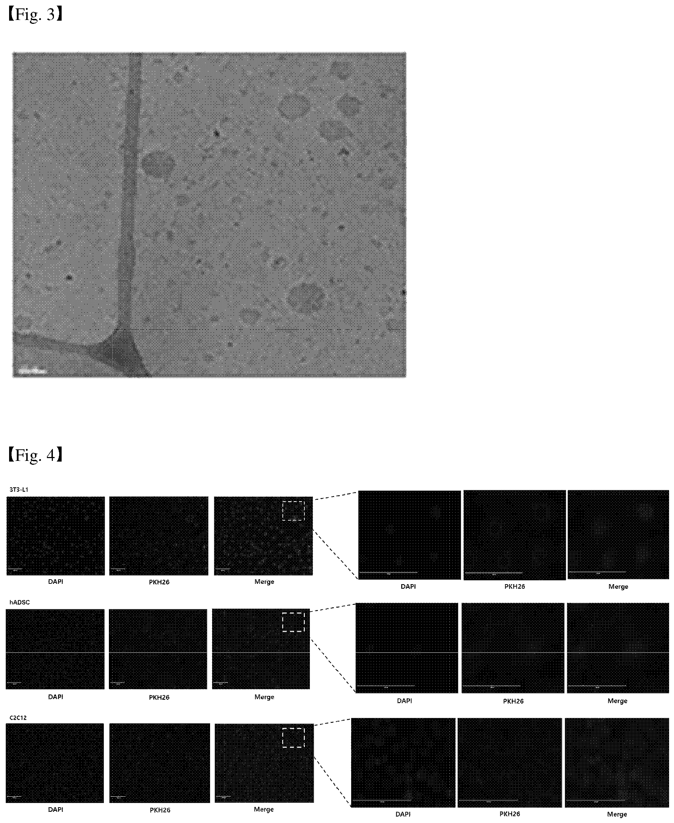

FIG. 3 shows the results of confirming the morphology and size of milk exosomes using an electron microscope.
FIG. 4 shows the results of confirming the intracellular uptake of milk exosomes.
FIG. 5 shows the results of confirming the effect of beige adipocyte differentiation by the browning induction effect of milk exosomes (A: 3T3-L1 cells, B: hADSC cells).
FIG. 6 shows the results of confirming the expression of UCP1 and PGC-la proteins by milk exosomes (A: 3T3-L1 cells, B: hADSC cells).

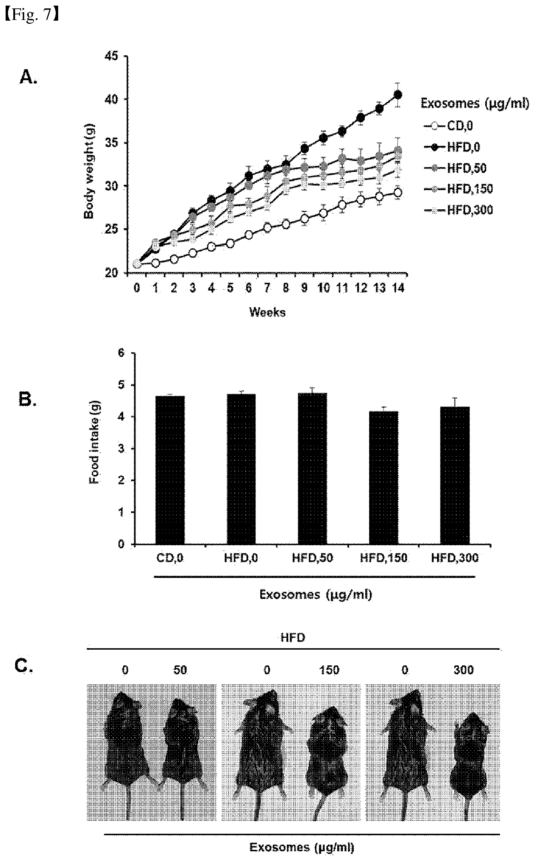

FIG. 7 shows the results of confirming the therapeutic effect of milk exosomes on obesity in an obesity mouse model with a high-fat diet (A: Confirmation of effect of body weight gain rate reduction, B: Confirmation of no significant change in food intake, and C: Confirmation of effect of body shape reduction).
FIG. 8A shows the results of confirming the expression of UCP1 and PGC-la proteins in iWAT by milk exosomes.
FIG. 8B shows the results of confirming the expression of lipolysis-related genes by milk exosomes.
FIG. 8C shows the results of confirming the expression of beige adipocyte-specific genes that appear through browning by milk exosomes.
FIG. 9 shows the results of confirming an increase in electron transport chain-related protein expression by milk exosomes (A: 3T3-L1 cells, B: hADSC cells).
FIG. 10 shows the results of confirming an increase in oxygen consumption rate by milk exosomes (A: 3T3-L1 cells, B: hADSC cells).
FIG. 11 shows the results of confirming the effect of ameliorating insulin resistance by milk exosomes (A: confirmation of blood glucose absorption capacity in 3T3-L1 cells, B: confirmation of blood glucose absorption capacity in C2C12 cells, C: confirmation of blood glucose production in primary hepatocytes, and D: confirmation of reduction in serum insulin).
FIG. 12 shows the results of confirming the mRNA expression of inflammatory factors and anti-inflammatory factors by milk exosomes (A: F4/80, B: TNF alpha, C: MCP1, D: IL-6, and E: IL-10).
FIG. 13 shows the results of confirming the effect of milk exosomes on reducing ALT levels.
FIG. 14 shows the results of confirming fat globules after administration of milk exosomes.
FIG. 15 shows the results of blood analysis after administration of milk exosomes (A: triglyceride, B: free fatty acid, C: HDL, and D: LDL).
FIG. 16 shows the results of confirming changes in the expression of UCP1 and PGC-la according to the overexpression of miRNA in milk exosomes (A: confirmation of mRNA expression, B: confirmation of protein expression).

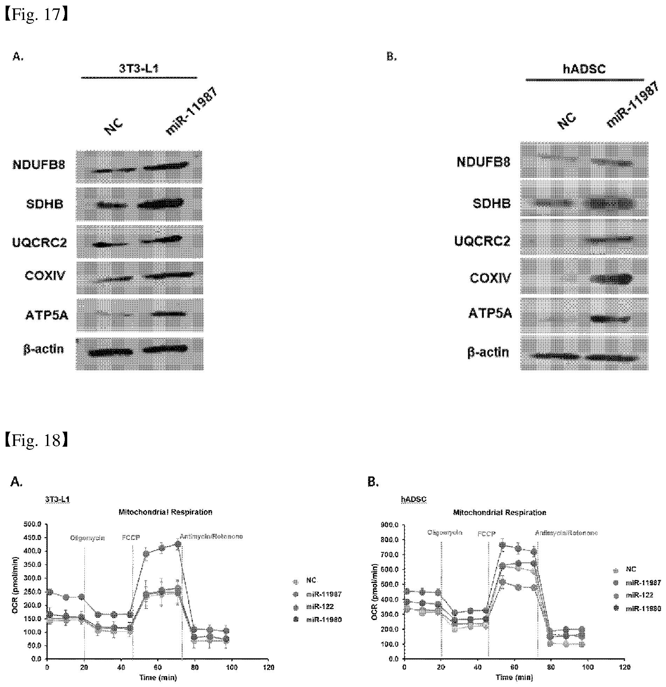

FIG. 17 shows the results of confirming an increase in the expression of electron transport chain-related proteins by miR-11987 overexpression (A: 3T3-L1 cells, B: hADSC cells).
FIG. 18 shows the results of confirming an increase in oxygen consumption rate by miR-11987 overexpression (A: 3T3-L1 cells, B: hADSC cells).

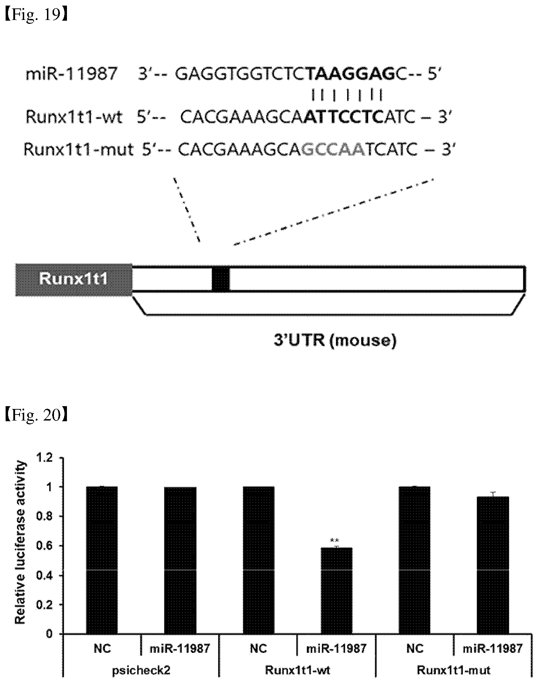

FIG. 19 shows a schematic view of the site of miR-11987 that binds to Runxltl.
FIG. 20 shows the results of confirming a decrease in Runxltl expression by miR-11987.

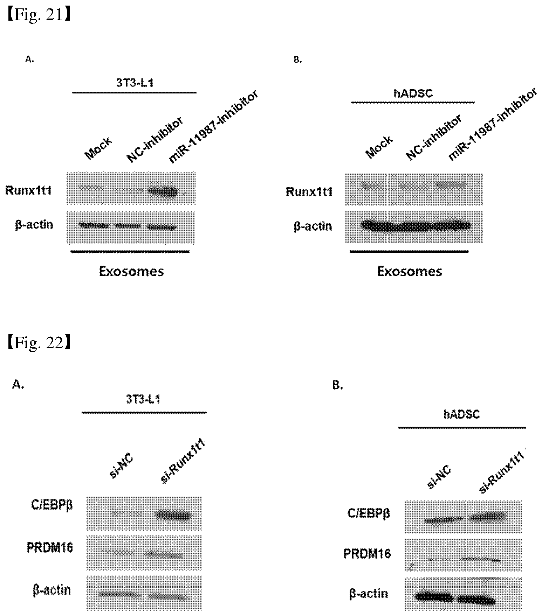

FIG. 21 shows the results of confirming a re-increase in the protein expression of Runxltl, which was decreased by milk exosomes by the treatment with miR-11987 inhibitor (A: 3T3-L1 cells, B: hADSC cells).
FIG. 22 shows the results of confirming an increase in the expression of C/EBPβ and PRDM16 proteins according to the introduction of Runxltl siRNA (A: 3T3-L1 cells, B: hADSC cells).

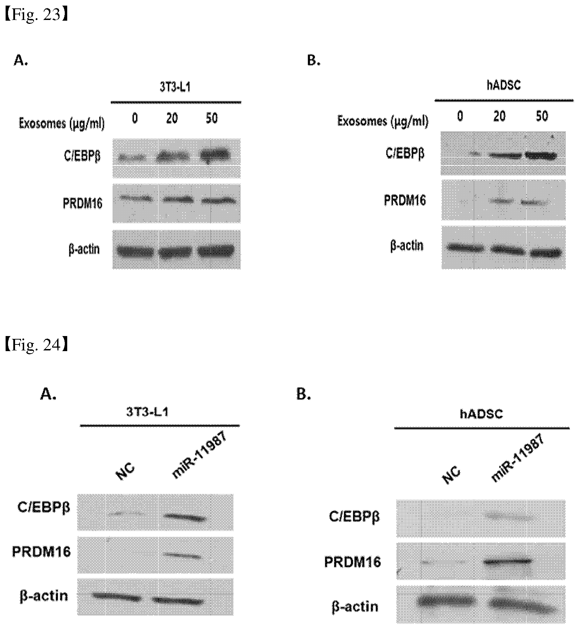

FIG. 23 shows the results of confirming an increase in the expression of C/EBPβ and PRDM16 proteins by milk exosomes (A: 3T3-L1 cells, B: hADSC cells).
FIG. 24 shows the results of confirming an increase in the expression of C/EBPβ and PRDM16 proteins by miR-11987 overexpression (A: 3T3-L1 cells, B: hADSC cells).

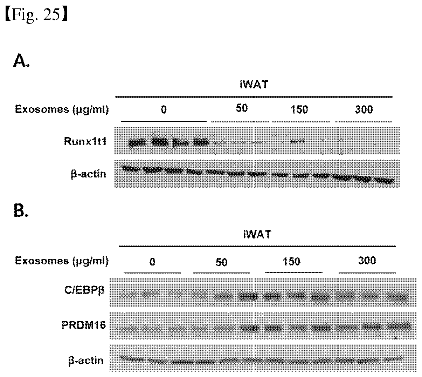

FIG. 25 shows the results of confirming the protein expression of Runxltl, C/EBPβ and PRDM16 by milk exosomes in iWAT (A: Runxltl, B: C/EBPβ and PRDM16).

### DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Example 1. Preparation of milk exosomes

Exosomes were extracted from cow-derived milk using a centrifuge. More specifically, milk was aliquoted into tubes, and the supernatant was collected by centrifugation at 2000 g and 10,000 g for 10 minutes, respectively. The collected supernatant was filtered through 0.45 µm and 0.2 µm filters, and then mixed with phosphate buffered saline (PBS). Thereafter, the Exoquick exosome precipitation solution (System Biosciences) was mixed with PBS, the resulting mixture was added thereto, and the resultant was allowed to stand for 30 minutes, and centrifuged again at 10,000 g for 10 minutes. The supernatant was removed, an exosome pellet was dissolved in PBS, the dissolved pellet was centrifuged again at 1,000 g for 1 minute, and then the supernatant was used for an experiment.

### Experimental Example 1: Confirmation of characteristics of milk exosomes

### 1-1. Measurement of size of milk exosomes

In order to confirm the size of the milk exosomes, the size of the exosomes was measured through dynamic light scattering (DLS), and it was confirmed that the size of the milk exosomes was within 200 nm (FIG. 1).

### 1-2. Confirmation of TSG101, CD9 and HSP70 protein expression

It was confirmed using western blot whether TSG101, CD9 and HSP70, which are milk exosome marker proteins, were expressed.

Proteins were isolated from milk exosomes using a 10% SDS polyacrylamide gel. After the isolated protein was transferred to a nitrocellulose membrane (GE Healthcare), it was reacted with a 5 % skim milk powder for 1 hour to prevent the non-specific binding of an antibody. Each primary antibody of TSG101 (Abcam), CD63 (Abcam) and HSP70 (Invitrogen) was diluted at a ratio of 1:1000 and bound to the membrane at 4°C for 12 to 18 hours. Thereafter, it was combined with an HRP-tagged anti-rabbit (KPL) antibody and reacted at room temperature for 30 minutes. Protein bands were observed using an ECL kit (Advansta Inc).

As a result, as showed in FIG. 2, it was confirmed that TSG101, CD9 and HSP70 proteins, which are the exosome marker proteins, were clearly observed in exosomes and not expressed in the supernatant other than exosomes.

### 1-3. Confirmation of morphology and size of exosomes

The morphology and size of the milk exosomes extracted in Example 1 were observed using an electron microscope. As a result, as showed in FIG. 3, it was confirmed that the exosomes were present in the form of round nanoparticles within a size of 200 nm.

### 1-4. Confirmation of intracellular uptake of exosomes

In order to confirm whether exosomes extracted from milk were absorbed into cells, the exosomes were treated with a PKH26 staining solution for 5 minutes, the stained exosomes were reacted with 3T3-L1 preadipocytes, adipose-derived mesenchymal stem cells (ADSCs) and C2C12 muscle cells for 3 hours, and then fluorescence was observed.

Specifically, 1 ml of Diluent C was added to the exosome pellet, and separately, a staining solution was produced by mixing 1 ml of Diluent C with 4 µl of PKH26 (Sigma). The staining solution was mixed with the exosomes to which Diluent C was added, the resulting mixture was reacted for 5 minutes, and the reaction was stopped while 1% bovine serum albumin was added. Exosomes labeled with PKH26 were extracted using an Exoquick exosome precursor solution (System Biosciences) according to the manufacturer's protocol. Thereafter, the extracted exosomes were observed using a fluorescence microscope (CELENA S digital imaging system, Logos Biosystems).

As a result, as showed in FIG. 4, it was confirmed that stained exosomes were contained in 3T3-L1 adipocytes, adipocyte-derived stem cells (hADSCs) and C2C12 muscle cells. Through this, it was confirmed that exosomes were extracted from milk, and intracellular uptake of the extracted exosomes appeared.

### Experimental Example 2. Confirmation of effect of milk exosomes on induction of browning of white fat

3T3-L1 cells and adipose-derived stem cells were aliquoted in a 6-well plate so as to be at 3×10⁵ cells/ml, and cultured in an incubator under 37°C and 5% CO2 conditions. When 3T3-L1 cells were saturated, the cells were cultured in a cell differentiation-inducing medium containing 10% fetal bovine serum, 1 µM dexamethasone, 10 µg/ml insulin and 0.5 mM 3-isobutyl-1-methylxanthine. After two days, the cultured cells were treated with 10% fetal bovine serum and 10 µg/ml insulin for 48 hours. Thereafter, the cells were cultured in a medium containing 10% fetal bovine serum for 2 days.

In addition, when adipose-derived stem cells were saturated, the cells were cultured while replacing a cell differentiation-inducing medium containing 10% fetal bovine serum, 1 µM dexamethasone, 10 µg/ml insulin, 0.5 mM 3-isobutyl-1-methylxanthine and 100uM indomethacin once every two days for 14 days.

Thereafter, the cells were treated with 20 µg/ml and 50 µg/ml of milk exosomes together with an adipose differentiation medium using a real-time polymerase chain reaction and western blot. That is, the cells were treated with milk exosomes once every two days during the induction of adipose differentiation. Thereafter, the effect of inducing browning of white fat was confirmed by confirming the expression of beige adipocyte-specific genes.

### 2-1. Confirmation of expression of beige adipocyte-specific genes

In order to confirm whether milk exosomes affect the browning of white fat, the expression of Cbp/P300 interacting transactivator with Glu/Asp rich carboxy-terminal domain 1 (CITED 1), heat shock protein family B (small) member 7 (HSPB7), TNF receptor superfamily member 9 (TNFRSF9), COUP TF-gamma, ERBAL2; NR2F6 (EAR2), CD40, EBF transcription factor 3 (EBF3), eva-1 homolog A (EVA1A) and pyruvate dehydrogenase kinase 4 (PDK4) genes, which are beige adipocyte-specific genes appearing through browning of beige fat, was confirmed by real-time PCR.

Specifically, 3T3-L1 and hADSC cell lines were each aliquoted on 6-well plates at 3×10⁵ cells/well, and then treated with milk exosomes along with a differentiation medium, and cells were collected after inducing differentiation of 3T3-L1 for 6 days and ADSC for 14 days. Total RNA was isolated by reacting the collected cells with Tri reagent (Bioline). In order to perform a reverse transcription reaction, dNTP and M-MLV reverse-transcriptase (Promega) were added to 1 µg of RNA, and then reacted at 37°C for 1 hour to synthesize cDNA. A real-time polymerase chain reaction was performed using SYBR Green PCR Master mix (Bioline) to measure CITED1, HSPB7, TNFRSF9, EAR2, CD40, EBF3, EVA1A and PDK4 mRNA expression. PCR conditions for amplifying a specific gene were performed as follows. After reaction at 95°C for 10 minutes, the gene was amplified for 40 cycles with a cycle of 5 seconds at 95°C, 10 seconds at 60°C, and 15 seconds at 72°C. A target gene mRNA expression level was corrected with the expression level relative to the actin expression level. The primers used are as shown in the following Table 1.

**[Table 1]**

| SEQ ID NO | Primer | Sequence (5'-3') |
|---|---|---|
| 1 | CITED 1 Forward (mouse) | 5'-AACCTTGGAGTGAAGGATCGC-3' |
| 2 | CITED 1 Reverse (mouse) | 5'-GTAGGAGAGCCTATTGGAGATGT-3' |
| 3 | HSPB7 Forward (mouse) | 5'-GAGCATGTTTTCAGACGACTTTG-3' |
| 4 | HSPB7 Reverse (mouse) | 5'-CCGAGGGTCTTGATGTTTCCTT -3' |
| 5 | TNFRSF9 Forward (mouse) | 5'-CGTGCAGAACTCCTGTGATAAC-3' |
| 6 | TNFRSF9 Reverse (mouse) | 5'-GTCCACCTATGCTGGAGAAGG-3' |
| 7 | TNFRSF9 Forward (human) | 5'-GGCAGGTGTAGCTGAGGTT-3' |
| 8 | TNFRSF9 Reverse (human) | 5'-GGACAGGGACTGCAAATCTGAT -3' |
| 9 | EAR2 Forward (mouse) | 5'-GAGGACGATTCGGCGTCAC-3' |
| 10 | EAR2 Reverse (mouse) | 5'-GTAATGCTTTCCACTGGACTTGT-3' |
| 11 | EAR2 Forward (human) | 5'-GAGCGGCAAGCATTACGGT-3' |
| 12 | EAR2 Reverse (human) | 5'-GGCAGGTGTAGCTGAGGTT-3' |
| 13 | CD40 Forward (mouse) | 5'-TGTCATCTGTGAAAAGGTGGTC-3' |
| 14 | CD40 Reverse (mouse) | 5'-ACTGGAGCAGCGGTGTTATG-3' |
| 15 | CD40 Forward (human) | 5'-ACTGAAACGGAATGCCTTCCT-3' |
| 16 | CD40 Reverse (human) | 5'-CCTCACTCGTACAGTGCCA-3' |
| 17 | EBF3 Forward (mouse) | 5'-TCACCCTCCCTTCAAACTGTA-3' |
| 18 | EBF3 Reverse (mouse) | 5'-GTTTCACTGCGGAGATGACAT-3' |
| 19 | EBF3 Forward (human) | 5'-AACAGGCCATCGTCTACGAG-3' |
| 20 | EBF3 Reverse (human) | 5'-GGCGTTTCGTTTCTATTGCCA-3' |
| 21 | EVA1A Forward (mouse) | 5'-GGGGAGACCGAAGGAAATGAGA-3' |
| 22 | EVA1A Reverse (mouse) | 5'-CTCCAGCCCTGCACACTCTA-3' |
| 23 | EVA1A Forward (human) | 5'-GCAAGACGCGAAACCTGAAC-3' |
| 24 | EVA1A Reverse (human) | 5'-TTCAAATCTGGGCTCGTCCC-3' |
| 25 | PDK4 Forward (mouse) | 5'-AGGGAGGTCGAGCTGTTCTC-3' |
| 26 | PDK4 Reverse (mouse) | 5'-GGAGTGTTCACTAAGCGGTCA-3' |
| 27 | β-actin Forward (mouse) | 5'-GTGACGTTGACATCCGTAAAGA-3' |
| 28 | β-actin Reverse (mouse) | 5'-GCCGGACTCATCGTACTCC-3' |
| 29 | β-actin Forward (human) | 5'-CATGTACGTTGCTATCCAGGC-3' |
| 30 | β-actin Reverse (human) | 5'-CTCCTTAATGTCACGCACGAT -3' |

As a result, as showed in FIG. 5A, it was confirmed that when preadipocytes 3T3-L1 cells were treated with milk exosomes, the expression of CITED 1, HSPB7, TNFRSF9, EAR2, CD40, EBF3, EVA1A and PDK4, which are beige adipocyte-specific genes appearing through browning, was all remarkably increased.

Furthermore, as showed in FIG. 5B, it was confirmed that when adipocyte-derived stem cells (hADSCs) were also treated with milk exosomes, the expression of TNFRSF9, EAR2, CD40, EBF3 and EVA1A, which are beige adipocyte-specific genes appearing through browning, was increased.

When cells were treated with milk exosomes, through changes in gene expression as described above, it was confirmed that the cells differentiated into beige adipocytes because browning was induced, and furthermore, it was confirmed that as the concentration of milk exosomes was increased, the induction of browning was increased.

### 2-2. Confirmation of UCP1 and PGC-la protein expression

In order to confirm whether milk exosomes affect the expression of the thermogenic genes UCP1 and PGC-la, the degree of protein expression of UCP1 and PGC-la was confirmed.

3T3-L1 and hADSC cell lines were each aliquoted on 6-well plates at 3×10⁵ cells/well, and then treated with milk exosomes along with a differentiation medium, and the cell lines were collected after inducing differentiation of 3T3-L1 for 6 days and ADSC for 14 days. After a supernatant was obtained by centrifuging the collected cells at 16,500 g for 15 minutes, the protein concentration was quantified using a BSA kit (Bio-Rad).

The extracted protein was isolated using a 10% SDS polyacrylamide gel. After the isolated protein was transferred to a nitrocellulose membrane (GE Healthcare), it was reacted with a 5% skim milk powder for 1 hour to prevent the non-specific binding of an antibody. The primary antibodies of uncoupling protein 1 (UCP1; Abcam), peroxisome proliferator-activated receptor gamma coactivator 1-alpha (PGC-1α; Booster Bio) and β-actin (Sigma) were diluted at a ratio of 1:1000 at 4°C, and reacted with a nitrocellulose membrane for 12 to 18 hours. Thereafter, after washing with PBS, an HRP-tagged anti-rabbit (KPL) antibody was added thereto and the resulting mixture was reacted at room temperature for 30 minutes. Protein bands were observed using an ECL kit (Santa Cruz Biotechnology).

As a result, as showed in FIG. 6, it was confirmed that when the cells were treated with milk exosomes, the protein expression of UCP1 and PGC-la was increased in both 3T3-L1 cells (Fig. 6A), which are preadipocytes, and hADSCs (Fig. 6B), which are adipose-derived stem cells, and it was confirmed that the protein expression was further increased as the concentration of the treated milk exosomes was increased. The aforementioned results indicate that the milk exosome of the present invention induces browning.

### Experimental Example 3. Confirmation of therapeutic effect of high-fat diet on obesity in obesity mouse model

### 3-1. Observation of body weight and appearance

In order to confirm the therapeutic effect of milk exosomes on obesity, C57BL6/J mice (male, 8 weeks old, Raonbio) were orally administered milk exosomes while being supplied with a high-fat diet (HFD) for 14 weeks (0 ug/ml (Control): 4 animals, 50 ug/ml exosomes: 3 animals, 100 ug/ml exosomes: 3 animals, and 300 ug/ml exosomes: 3 animals).

Specifically, after the exosomes were administered at a concentration of 50, 150, and 300 µg/ml for 14 weeks, a change in body weight was measured, and PBS containing no exosomes was used for the negative control group. After 14 weeks, a change in body weight and appearance were observed to confirm the therapeutic effect on obesity.

As a result, as showed in FIG. 7A, when milk exosomes were administered, the body weight gain rate was decreased, but as showed in FIG. 7B, there was no difference in food intake according to the administration of milk exosomes. In particular, the body weight gain rate was further decreased as the administration concentration of milk exosomes was increased, and from FIGS. 7A and 7B, it was confirmed that the administration of milk exosomes could show the effect of reducing the body weight gain rate even without a decrease in food intake.

Furthermore, as showed in FIG. 7C, it was confirmed that when milk exosomes were administered, the body size was also decreased, and thus, the body size appeared smaller.

### 3-2. Confirmation of protein expression

After the high-fat diet obesity mouse model was euthanized, an inguinal white adipose tissue (iWAT), which is an inguinal region adipose tissue, was isolated by laparotomy.

Thereafter, the protein expression levels of UCP1 and PGC-la in the iWAT were confirmed by western blot. The western blotting method is as described in 2-2 above.

The number of lanes in FIG. 8A is the same as the number of experimental groups from which iWAT tissue was collected (0 ug/ml exosomes (control): 4 animals, mice ingesting 50 ug/ml exosomes: 3 animals, mice ingesting 100 ug/ml exosomes: 3 animals, and mice ingesting300 ug/ml exosomes: 3 animals).

As a result, as showed in FIG. 8A, it was confirmed that the protein expression of the thermogenic genes UCP1 and PGC-la was increased when milk exosomes were administered, and it was confirmed that the protein expression of UCP1 and PGC-la was increased as the concentration of the administered milk exosomes was increased.

### 3-3. Confirmation of gene expression

The expression of lipolysis-related genes and beige adipocyte-specific genes in the isolated iWAT was confirmed using a real-time polymerase chain reaction. The real-time polymerase chain reaction is as described in 2-1 above, and the primer sequences used for lipolysis-related genes are summarized in the following Table 2.

**[Table 2]**

| SEQ ID NO | Primer | Sequence (5'-3') |
|---|---|---|
| 31 | HSL Forward | 5'-ATGGATTTACGCACGATGACA-3' |
| 32 | HSL Reverse | 5'-TAGCGTGACATACTCTTGCAG-3' |
| 33 | ATGL Forward | 5'-CAACGCCACTCACATCTACGG-3' |
| 34 | ATGL Reverse | 5'-GGACACCTCAATAATGTTGGCAC-3' |

As a result, as showed in FIG. 8B, it was confirmed that when milk exosomes were administered, the expression of hormone-sensitive lipase (HSL), which is a lipolytic enzyme, and adipose triglyceride lipase (ATGL), which is a triglyceride lipase, was increased. In particular, it was confirmed that the expression of HSL and ATGL was further increased as the administration concentration of milk exosomes was increased.

Further, as showed in FIG. 8C, it was confirmed that when milk exosomes are administered, the expression of CITED1, HSPB7, TNFRSF9, EAR2, CD40, EBF3, EVA1A and PDK4, which are beige adipocyte-specific genes appearing through browning, was all remarkably increased, and furthermore, it was confirmed that as the concentration of milk exosomes was increased, the induction of browning was increased.

The aforementioned results suggest that administration of the milk exosome of the present invention can show a therapeutic effect on obesity and a therapeutic effect on metabolic diseases caused by obesity through the treatment of obesity.

### Experimental Example 4. Confirmation of mitochondrial activity effect of milk exosomes

In order to confirm the effect of milk exosomes on mitochondrial activity, the expression and oxygen consumption rate of electron transport chain-related proteins were confirmed.

### 4-1. Confirmation of increase in expression of electron transport chain-related proteins

Changes in the expression of NADH:ubiquinone oxidoreductase subunit B8 (NDUFB8), succinate dehydrogenase complex iron sulfur subunit B (SDHB), ubiquinol-cytochrome C reductase core protein 2 (UQCRC2), cytochrome c oxidase subunit IV (COX IV) and ATP synthase F1 subunit alpha, mitochondrial (ATP5A) as electron transport chain-related proteins were confirmed.

3T3-L1 and hADSC cell lines were each aliquoted on 6-well plates at 3×10⁵ cells/well, and then treated with milk exosomes along with a differentiation medium, and the differentiation of 3T3-L1 and ADSC was induced for 6 days and 14 days, respectively. Thereafter, the cells were collected, western blot was performed, and the western blotting method is as described in 2-2 above. As a primary antibody in the present western blot, antibodies against NDUFB8 (Invitrogen), SDHB (Invitrogen), UQCRC2 (Abcam), COXIV (Abcam) and ATP5A (Invitrogen) were used.

As a result, as showed in FIG. 9, it was confirmed that when both 3T3-L1 cells and hADSC cells were treated with milk exosomes, the expression of proteins involved in electron transport chain activity in mitochondria was increased (FIGS. 9A and 9B).

In addition, iWAT, which is an adipose tissue, was obtained from the obesity model mouse to confirm proteins expressed in the iWAT tissue by the same western blotting method (FIG. 9C). The number of lanes in FIG. 9C is the same as the number of experimental groups from which iWAT tissue was collected (Control: 4 animals, mice ingesting 50 ug/ml exosomes: 3 animals, mice ingesting 100 ug/ml exosomes: 3 animals, and mice ingesting 300 ug/ml exosomes: 3 animals).

As showed in FIG. 9C, it was confirmed that when iWAT tissue was also treated with milk exosomes, the expression was increased (FIG. 9C).

### 4-2. Confirmation of increase in oxygen consumption rate

3T3-L1 and hADSC cell lines were each aliquoted on 24 multi well plates (Seahorse Bioscience) at 5×10⁴ cells/well, and then treated with milk exosomes along with a differentiation medium, and the differentiation of 3T3-L1 and ADSC was induced for 6 days and 14 days, respectively. Thereafter, cells were sequentially treated with 1.5 µM oligomycin, 0.75 µM carbonylcyanide-4-(trifluoromethoxy)phenylhydrazone (FCCP), 1 µM rotenone and antimycin, and then the oxygen consumption amount of the cells was measured using an XF analyzer (Seahorse).

As a result, it was confirmed that the oxygen consumption rate increased when both 3T3-L1 cells (FIG. 10A) and hADSCs (FIG. 10B) were treated with milk exosomes.

### Experimental Example 5. Confirmation of insulin resistance amelioration effect of milk exosomes

In order to confirm whether milk exosomes ameliorate insulin resistance, blood glucose absorption capacity was observed after 3T3-L1 adipocytes and C2C12 muscle cells were treated with milk exosomes.

3T3-L1 and C2C12 cell lines were each aliquoted on 6-well plates at 3×10⁵ cells/well, and then treated with milk exosomes along with a differentiation medium, and the adipose differentiation of 3T3-L1 was induced for 6 days. After C2C12 cells were saturated, muscle differentiation was performed for 6 days while replacing a medium containing 2% horse serum (Gibco) once every 2 days. Glucose uptake was measured using a 2-deoxyglucose uptake measurement kit (Cosmo Bio) after treating cells with 1 µM insulin and 1 mM 2-deoxyglucose.

As a result, as showed in FIGS. 11A and 11B, it was confirmed that when both 3T3-L1 adipocytes and C2C12 muscle cells were treated with milk exosomes, intracellular blood glucose uptake increased.

Furthermore, blood glucose production was confirmed using primary hepatocytes.

Specifically, livers extracted from mice were cultured in Medium 199 (Gibco) containing 5% fetal bovine serum (FBS) and penicillin/streptomycin (100 µg/ml) in collagen I 6 well plates for 24 hours. The livers were cultured in a FBS-deficient Medium 199 medium for 16 to 18 hours and then washed with PBS. Thereafter, the livers were treated with 0.1 mm pCPT-cAMP and cultured in an incubator at 37°C for 6 hours, and then glucose production was measured using a colorimetric glucose assay kit (BioVision).

As a result, as showed in FIG. 11C, it was confirmed that when primary hepatocytes were treated with milk exosomes, blood glucose production was reduced.

Further, insulin metabolism was confirmed. Specifically, blood was collected from mice on a normal diet and mice on a high-fat diet. Blood was mixed well and left to stand, and then centrifuged at 3000 rpm for about 20 minutes after 30 minutes had passed to isolate serum. The serum was used to measure an insulin metabolic rate using a mouse insulin ELISA kit (ALPCO).

As a result, as showed in FIG. 11D, it was confirmed that serum insulin was decreased when serum was treated with milk exosomes.

The aforementioned results indicate that the milk exosome of the present invention improves insulin metabolism.

### Experimental Example 6. Effect of milk exosomes on ameliorating metabolic dysfunction due to obesity

### 6-1. Confirmation of decrease in expression of inflammatory and anti-inflammatory-related genes

In order to confirm the therapeutic effect of milk exosomes on obesity, C57BL6/J mice (male, 8 weeks old, Raonbio) were orally administered milk exosomes while being supplied with a high-fat diet (HFD) for 14 weeks (0 ug/ml (Control): 4 animals, 50 ug/ml exosomes: 3 animals, 100 ug/ml exosomes: 3 animals, and 300 ug/ml exosomes: 3 animals).

Specifically, after mice administered the milk exosomes at a concentration of 50, 150 and 300 µg/ml for 14 weeks were euthanized, mRNA expression of inflammatory and anti-inflammatory related factors was confirmed by opening the abdomen to isolate epididymal white adipose tissue (eWAT), which is an epididymal adipose tissue.

The mRNA expression of F4/80, TNF alpha, MCP1 and IL-6 as inflammatory-related factors was confirmed, and the gene expression of IL-10 as an anti-inflammatory-related factor was confirmed. They were confirmed using a real-time polymerase chain reaction. The real-time polymerase chain reaction is as described in 2-1 above, and the primer sequences used are summarized in the following Table 3.

**[Table 3]**

| SEQ ID NO | Primer | Sequence (5'-3') |
|---|---|---|
| 35 | F4/80 Forward | 5'-CCCCAGTGTCCTTACAGAGTG-3' |
| 36 | F4/80 Reverse | 5'-GTGCCCAGAGTGGATGTCT-3' |
| 37 | TNF-alpha Forward | 5'-GACGTGGAACTGGCAGAAGAG-3' |
| 38 | TNF-alpha Reverse | 5'-TTGGTGGTTTGTGAGTGTGAG-3' |
| 39 | MCP1 Forward | 5'-AGGTGTCCCAAAGAAGCTGT-3' |
| 40 | MCP1 Reverse | 5'-AAGACCTTAGGGCAGATGCAG-3' |
| 41 | IL-6 Forward | 5'-ACAAGTCCGGAGAGGAGACT-3' |
| 42 | IL-6 Reverse | 5'-TGTGACTCCAGCTTATCTCTTGG-3' |
| 43 | IL-10 Forward | 5'-GCTCTTGCACTACCAAAGCC-3' |
| 44 | IL-10 Reverse | 5'-CTGCTGATCCTCATGCCAGT -3' |

As a result, as showed in FIG. 12, it was confirmed that when milk exosomes were administered, the mRNA expression of the inflammatory factors F4/80, TNF alpha, MCP1 and IL-6 was decreased, and the mRNA expression of the anti-inflammatory factor IL-10 was increased.

### 6-2. Confirmation of ALT reduction

Blood was collected from mice on a high-fat diet. Blood was mixed well and left to stand, and then centrifuged at 3,000 rpm for about 20 minutes after 30 minutes had passed to isolate serum. Serum was measured using an ALT assay kit (Cusabio).

As a result, as showed in FIG. 13, it was confirmed that the level of ALT, which is a marker of fatty liver, was decreased in a mouse serum administered milk exosomes. In particular, the ALT level was decreased at a concentration of 150 µg/ml or higher.

### 6-3. Confirmation of fat globules

After the liver was collected from mice on a high-fat diet, 5 µm-sections were produced by slicing a paraffin-embedded liver sample. The sections were deparaffinized with a deparaffinization solution, a lysis buffer was added, and then the sections were stained with hematoxylin and eosin.

As a result, as showed in FIG. 14, it could be seen that milk exosomes caused less fat to appear in the livers of mice administered milk exosomes, with smaller numbers of smaller fat globules appearing than in the livers not administered milk exosomes.

### 6-4: Blood analysis

Blood was collected from mice on a high-fat diet. Blood was mixed well and left to stand, and then centrifuged at 3,000 rpm for about 20 minutes after 30 minutes had passed to isolate serum. Serum was measured using a free fatty acid assay kit (Abcam), a cholesterol assay kit (Abcam), and a triglyceride quantification assay kit (Abcam).

As a result, as showed in FIG. 15, it was confirmed that when milk exosomes were administered, triglyceride (FIG. 15A), free fatty acid (FIG. 15B), and LDL (FIG. 15D) were significantly reduced, and HDL (FIG. 15C) was significantly increased.

### Experimental Example 7. Confirmation of microRNA (miRNA) in milk exosomes

RNA-seq was performed to confirm the microRNA present in milk exosomes, and among these genes, miR-11987, miR-122, miR-11980, miR-21, miR-1777b miR-2478, miR-92a, miR-1777a and miR-2430 with a read-count of 2000 or higher were selected.

In order to confirm whether the white fat was browned by the selected 9 microRNAs, 9 microRNAs were overexpressed in 3T3-L1 adipocytes, and then the mRNA expression of UCP1 and PGC-la was compared.

3T3-L1 cells were aliquoted in a 6-well plate so as to be at 3×10⁵ cells/ml, and cultured in an incubator under 37°C and 5% CO2 conditions. 9 microRNAs were overexpressed in 3T3-L1 cells using Lipofectamine 2000. Thereafter, the cells were cultured in a cell differentiation-inducing medium containing 10% fetal bovine serum, 1 µM dexamethasone, 10 µg/ml insulin and 0.5 mM 3-isobutyl-1-methylxanthine. After two days, the cultured cells were treated with 10% fetal bovine serum and 10 µg/ml insulin for 48 hours. Thereafter, the cells were cultured in a medium containing 10% fetal bovine serum for 2 days, and then the cells were collected.

The gene expression measurements of UCP1 and PGC-la were confirmed using a real-time polymerase chain reaction. The real-time polymerase chain reaction is as described in 2-1 above, and the primer sequences used are summarized in the following Table 4.

**[Table 4]**

| SEQ ID NO | Primer | Sequence (5'-3') |
|---|---|---|
| 45 | UCP1 Forward | 5'-AGGCTTCCAGTACCATTAGGT-3' |
| 46 | UCP1 Reverse | 5'-CTGAGTGAGGCAAAGCTGATTT-3' |
| 47 | PGC-1α Forward | 5'-TATGGAGTGACATAGAGTGTGCT-3' |
| 48 | PGC-1α Reverse | 5'-CCACTTCAATCCACCCAGAAAG-3' |

In addition, the protein expression of UCP1 and PGC-la was confirmed using western blot, and the western blotting method was the same as described in Example 2-2.

As a result, as showed in FIG. 16, it was confirmed that when cells were treated with miR-11987, miR-122, and miR-11980 among the 9 microRNAs, both the mRNA (FIG. 16A) and protein (FIG. 16B) expression levels of UCP1 and PGC-1 α were increased. The sequences of miR-11987, miR-122 and miR-11980 are summarized in the following Table 5.

**[Table 5]**

| SEQ ID NO | Primer | Sequence (5'-3') |
|---|---|---|
| 49 | miR-11987 | 5'-cgaggaaucucugguggaggu-3' |
| 50 | miR-122 | 5'-uggagugugacaaugguguuug-3' |
| 51 | miR-11980 | 5'-aggcaacgggcuuggcggag-3' |

### Experimental Example 8. Confirmation of mitochondrial activity effect of miR-11987

After miR-11987 was overexpressed in each of 3T3-L1 cells and hADSC cells, mitochondrial activity was confirmed using the same method as in Experimental Example 4. The overexpression method of miR-11987 is the same as the method described in Experimental Example 7.

### 8-1. Confirmation of increase in expression of electron transport chain-related proteins

The expression of electron transport chain-related proteins was confirmed using the same method as in 4-1 above.

As a result, as showed in FIG. 17, it was confirmed that when both 3T3-L1 cells and hADSC cells were treated with milk exosomes, the expression of proteins involved in electron transport chain activity in mitochondria was increased (FIGS. 17A and 17B).

### 8-2. Confirmation of increase in oxygen consumption rate

The oxygen consumption rate was confirmed using the same method as in 4-2 above.

As a result, it was confirmed that when miR-11987 was overexpressed, the oxygen consumption rate was increased by treating both 3T3-L1 cells (FIG. 18A) and hADSCs (FIG. 18B) with milk exosomes.

### Experimental Example 9. Confirmation of target gene of miR-11987

### 9-1. Confirmation of Runx1t1 binding site, which is target gene of miR-11987

Intracellular genes affected by miR-11987 were investigated. Specifically, genes present in mice and humans that bind to miR-11987 were investigated using miRDB, which is a program that can predict the target gene and binding site of microRNA, and as a result, RUNX1 Partner Transcriptional Co-Repressor 1 (Runxltl) was selected.

In the Runx1t1 gene, a site that reacts with miR-11987 is conserved with other species, and the Runx1t1 gene suppresses the expression of C/EBPβ and PRDM16, which are regulators that induce brown/beige adipocytes. In order to investigate whether miR-11987 directly regulates the Runx1t1 gene, a site where miR-11987 reacts with Runx1t1 was first investigated, and a plasmid in which the reactive site was mutated was produced.

Specifically, a recombinant vector (psiCHECK2-Runx1t1-wt) was constructed by inserting a Runx1t1 fragment containing a miR-11987-binding site into a psiCHECK2 vector. Thereafter, a Runx1t1 site that binds to miR-11987 (psiCHECK2-Runx1t1-mut) was mutated using a QuickChange site-directed mutagenesis kit (Stratagene).

A schematic view of the site of miR-11987 that binds to Runx1t1 is as showed in FIG. 19.

### 9-2. Confirmation of expression regulation of miR-11987 for Runx1t1

A luciferase reporter analysis was performed to investigate whether Runx1t1 was regulated by miR-11987.

A Cos7 cell line was aliquoted at 3×10⁴ cells/well and cultured in 24-well plates for 24 hours. The constructed psiCHECK2-Runx1t1 (50ng) and a miR-11987 mimic (50nM) (Genepharma) were injected into cells using Lipofectamine 2000 (Invitrogen) to induce transformation. After transformed cells were cultured for 2 days, luciferase activity was measured with a dual luciferase assay kit (Promega) using a TD-20/20 luminometer (Turner Biosystems).

As a result, as showed in FIG. 20, it was confirmed that in the cells into which miR-11987 was introduced, Runxltl significantly reduced the luciferase activity to about 48%, and in the plasmid in which Runx1t1 reacting with miR-11987 was mutated, there was no difference from the control.

Furthermore, after an NC-inhibitor (GenePharma catalog number B04003) and a miR-11987 inhibitor (GenePharma catalog number B03001) were put into 50 µg/ml milk exosomes at a concentration of 50 nM in each of 3T3-L1 cells and hADSC cells into which miR-11987 was introduced by using sonicator, the cells were treated with a differentiation medium and cultured for 6 days. When miR-11987 was suppressed, the protein expression of Runxltl was confirmed. The cells were treated with the NC-inhibitor as a control reagent.

As a result, as showed in FIG. 21, it was confirmed that when the cells were treated with the miR-11987 inhibitor, the protein expression of Runx1t1 reduced by milk exosomes was again increased in both 3T3-L1 cells (FIG. 21A) and hADSC cells (FIG. 21B). From this, it was confirmed that miR-11987 directly suppresses the Runx1t1 gene.

### Experimental Example 10. Confirmation of increase in expression of C/EBPβ and PRDM16

### 10-1. Production and confirmation of Runx1t1 siRNA

Runxltl and negative control (NC) siRNA were purchased from GEpharma, and the sequences of Runx1t1 siRNA (si-Runx1t1) and NC siRNA (si-NC) are summarized in the following Table 6. The siRNA was introduced into cells according to the instructions of GEpharma.

**[Table 6]**

| SEQ ID NO | Gene | Direction | Sequence (5'-3') |
|---|---|---|---|
| 52 | Runx1t1 (mouse) | Sense | 5'-GUGGAAACAUCUCGAUCAUCU-3' |
| 53 | Runx1t1 (mouse) | Antisense | 5'-AGAUGAUCGAGAUGUUUCCAC-3' |
| 54 | Runx1t1 (human) | Sense | 5'-GGCGAUCUCUCACCGUACUAA-3' |
| 55 | Runx1t1 (human) | Antisense | 5'-UUAGUACGGUGAGAGAUCGCC-3' |
| 56 | NC (mouse) | Sense | 5'-UUUUCCGAACGUGUCACGUTT-3' |
| 57 | NC (mouse) | Antisense | 5'-ACGUGACACGUUCGGAGAATT-3' |
| 58 | NC (human) | Sense | 5'-UUUUCCGAACGUGUCACGUTT-3' |
| 59 | NC | Antisense | 5'-ACGUGACACGUUCGGAGAATT-3' |
| | (human) | | |

### 10-2. Confirmation of increase in expression of C/EBPβ and PRDM16 proteins by introducing Runx1t1 siRNA

After Runx1t1 siRNA (si-Runx1t1) produced in 10-1 above was introduced into each of 3T3-L1 cells and hADSC cells, it was confirmed by western blot whether the expression of C/EBPβ and PRDM16 was increased.

Specifically, 3T3-L1 cells and hADSC were each aliquoted into 6-well plates at 3×10⁵ cells/ml, and si-Runx1t1 was injected into cells using Lipofectamine 2000. Thereafter, the cells were cultured for 6 days using an adipose differentiation medium. Thereafter, the cells were collected, western blot was performed, and the western blotting method is as described in 2-2 above. As a primary antibody in the present western blot, antibodies against C/EBPβ (Abcam) and PRDM16 (R&D Systems) were used.

As a result, as showed in FIG. 22, it was confirmed that when Runxltl siRNA was introduced into both 3T3-L1 cells (FIG. 22A) and hADSC cells (FIG. 22B), the expression of C/EBPβ and PRDM16 proteins was increased.

### 10-3. Confirmation of increase in expression of C/EBPβ and PRDM16 proteins according to treatment with milk exosomes

3T3-L1 and hADSC cell lines were each aliquoted on 6-well plates at 3×10⁵ cells/well, and then treated with milk exosomes along with a differentiation medium, and the cell lines were collected after inducing differentiation of 3T3-L1 for 6 days and ADSC for 14 days. The expression of C/EBPβ and PRDM16 proteins was confirmed in the collected cells.

As a result, as showed in FIG. 23, it was confirmed that when the cells were treated with milk exosomes, the expression of C/EBPβ and PRDM16 proteins was increased in both 3T3-L1 cells (FIG. 23A) and hADSC cells (FIG. 23B), and the expression of C/EBPβ and PRDM16 proteins was further increased as the concentration of the milk exosomes was increased.

### 10-4. Confirmation of increase in expression of C/EBPβ and PRDM16 according to miR-11987 overexpression

After miR-11987 was overexpressed in each of 3T3-L1 cells and hADSC cells in the same manner as in Experimental Example 7, the expression of C/EBPβ and PRDM16 proteins was confirmed.

As a result, as showed in FIG. 24, it was confirmed that when miR-11987 was overexpressed, the expression of C/EBPβ and PRDM16 proteins was increased in both 3T3-L1 cells (FIG. 24A) and hADSC cells (FIG. 24B).

### Experimental Example 11. Confirmation of reduction in expression of Runxltl by milk exosomes

Milk exosomes were orally administered to C57BL6 /J mice (male, 8 weeks old, Raonbio) once every 3 days for 14 weeks, and iWAT, which is adipose tissue in the inguinal region, was isolated by euthanizing the mice, and then opening the abdomen.

Milk exosomes were administered at a concentration of 50, 150, and 300 µg/ml (0 ug/ml (control): 4 animals, 50 ug/ml exosomes: 3 animals, 100 ug/ml exosomes: 3 animals, and 300 ug/ml exosomes: 3 animals).

Thereafter, the protein expression levels of Runx1t1 (Proteintech), C/EBPβ and PRDM16 by milk exosomes in the iWAT were confirmed by western blot. The western blotting method is as described in 10-2 above.

As a result, as showed in FIG. 25, it was confirmed that the expression of Runxltl was reduced by milk exosomes even in the adipose tissue of mice (FIG. 25A), and conversely, the expression levels of C/EBPβ and PRDM16 were increased (FIG. 25B).

The aforementioned results indicate that milk exosomes induce browning of white fat by increasing the expression of C/EBPβ and PRDM16.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive. For example, each constituent element which is described as a singular form may be implemented in a distributed form, and similarly, constituent elements which are described as being distributed may be implemented in a combined form.

The scope of the present invention is represented by the following claims, and it should be interpreted that the meaning and scope of the claims and all the changes or modified forms derived from the equivalent concepts thereof fall within the scope

## Claims

1. A composition for inducing browning, comprising milk exosomes.

2. The composition of claim 1, wherein the milk exosomes comprise one or more miRNAs selected from the group consisting of miR-11987, miR-122 and miR-11980.

3. The composition of claim 2, wherein the miR-11987 comprises a base sequence of SEQ ID NO: 49,
the miR-122 comprises a base sequence of SEQ ID NO: 50, and
the miR-11980 comprises a base sequence of SEQ ID NO: 51.

4. The composition of claim 1, wherein the milk exosomes are derived from cows.

5. The composition of claim 1, wherein the milk exosomes increase the expression of uncoupling protein-1 (UCP-1) or peroxisome proliferator-activated receptor gamma coactivator 1-alpha (PGC-1α).

6. A pharmaceutical composition for preventing or treating obesity or a metabolic disease, comprising milk exosomes.

7. The pharmaceutical composition of claim 6, wherein the milk exosomes comprise one or more miRNAs selected from the group consisting of miR-11987, miR-122 and miR-11980.

8. The pharmaceutical composition of claim 7, wherein the miR-11987 comprises a base sequence of SEQ ID NO: 49,
the miR-122 comprises a base sequence of SEQ ID NO: 50, and
the miR-11980 comprises a base sequence of SEQ ID NO: 51.

9. The pharmaceutical composition of claim 6, wherein the metabolic disease is one or more selected from the group consisting of diabetes, hyperlipidemia, hypercholesterolemia, arteriosclerosis and fatty liver.

10. A food composition for ameliorating obesity or a metabolic disease, comprising milk exosomes.

11. A method for inducing differentiation of white adipocytes into beige adipocytes or brown adipocytes, the method comprising treating white adipocytes with milk exosomes.

12. The method of claim 11, wherein the milk exosomes comprise one or more miRNAs selected from the group consisting of miR-11987, miR-122 and miR-11980.

13. The method of claim 12, wherein the miR-11987 comprises a base sequence of SEQ ID NO: 49,
the miR-122 comprises a base sequence of SEQ ID NO: 50, and
the miR-11980 comprises a base sequence of SEQ ID NO: 51.

14. A method for treating obesity or a metabolic disease, the method comprising administering milk exosomes to an individual.

15. The method of claim 14, wherein the milk exosomes comprise one or more miRNAs selected from the group consisting of miR-11987, miR-122 and miR-11980.

16. The method of claim 15, wherein the miR-11987 comprises a base sequence of SEQ ID NO: 49,
the miR-122 comprises a base sequence of SEQ ID NO: 50, and
the miR-11980 comprises a base sequence of SEQ ID NO: 51.

17. The method of claim 14, wherein the metabolic disease is one or more selected from the group consisting of diabetes, hyperlipidemia, hypercholesterolemia, arteriosclerosis and fatty liver.
